# EUROPEAN PATENT SPECIFICATION

(11) **EP 4 240 472 B1**
(45) Date of publication and mention of the grant of the patent: **28.05.2025**
(21) Application number: 22704816.2
(22) Date of filing: 18.01.2022
(51) Int. Cl.: A61N 1/05

(54) **ELECTRICAL STIMULATION CUFF DEVICES AND SYSTEMS WITH DIRECTIONAL ELECTRODE CONFIGURATIONS**
MANSCHETTE ZUR ELEKTRISCHEN STIMULATION UND SYSTEME MIT GERICHTETEN ELEKTRODENKONFIGURATIONEN
DISPOSITIFS ET SYSTÈMES DE MANCHON DE STIMULATION ÉLECTRIQUE AYANT DES CONFIGURATIONS D'ÉLECTRODE DIRECTIONNELLES

(30) Priority: 19.01.2021 US 202163139240 P
(43) Date of publication of application: 13.09.2023
(73) Proprietor: Boston Scientific Neuromodulation Corporation, Valencia, CA 91355 (US)
(72) Inventor: SUBRAMANIAN, Hari, Hara, Valencia, CA 91355 (US)
(74) Representative: Vossius & Partner Patentanwälte Rechtsanwälte mbB
(86) International application number: PCT/US2022/012743
(87) International publication number: WO 2022/159375

(56) References cited:
- US-A1- 2003 040 785
- US-A1- 2014 046 407
- US-A1- 2018 318 578
- US-A1- 2020 384 265
- US-B2- 7 996 092

## Description

### FIELD

The present disclosure is directed to the area of implantable electrical stimulation systems and methods of making and using the systems. The present disclosure is also directed to implantable electrical stimulation cuff devices, as well as methods of making and using the same.

### BACKGROUND

Implantable electrical stimulation systems have proven therapeutic in a variety of diseases and disorders. For example, spinal cord stimulation systems have been used as a therapeutic modality for the treatment of chronic pain syndromes. Peripheral nerve stimulation has been used to treat chronic pain syndrome and incontinence, with a number of other applications under investigation. Functional electrical stimulation systems have been applied to restore some functionality to paralyzed extremities in spinal cord injury patients. Stimulation of the brain, such as deep brain stimulation, can be used to treat a variety of diseases or disorders.

Stimulators have been developed to provide therapy for a variety of treatments. A stimulator can include a control module (with a pulse generator), one or more leads, and an array of stimulator electrodes on each lead. The stimulator electrodes are in contact with or near the nerves, muscles, or other tissue to be stimulated. The pulse generator in the control module generates electrical pulses that are delivered by the electrodes to body tissue.

US 2003/040785 A1 discloses an electrode assembly to be installed on a patient's nerve which has a circum-neural carrier with a split circumferential configuration longitudinally attached to a lead at the distal end thereof.

US 7 996 092 B2 describes devices, systems, and methods for recording, and/or stimulation, and/or blocking of a nerve make use of a molded nerve cuff electrode. One or more electrodes are positioned in one or more frames within a casing of the cuff electrode.

US 2020/384265 A1 discloses a nerve stimulation system including at least one nerve interface device. The device includes a cuff portion having an assembled position in which the cuff portion forms at least part of a passageway for receiving a nerve along a longitudinal axis passing through the passage-way; and first and second rings of electrodes mounted on the cuff portion, each ring of electrodes including a plurality of electrodes, and wherein each electrode in the first ring has a corresponding longitudinally-aligned electrode in the second ring so as to form a plurality of pairs of electrodes spaced apart from each other along the longitudinal axis.

US 2014/046407 A1 discloses an electrode device configured to be coupled to a parasympathetic site of a subject. A control unit is configured to drive the electrode device to apply a current in bursts of one or more pulses.

US 2018/318578 A1 describes an electrode lead comprising an elongated lead body, at least one lead connector terminal affixed to the proximal end of the lead body, and an electrically insulative cuff body affixed to the distal end of the lead body. The cuffbody is configured for being circumferentially disposed around a nerve. The cuffbody comprises cutouts, slits, a wrinkled portion, a thin stretchable portion, and/or a serpentine strap, which increases that increase the expandability of the cuff body when disposed around the nerve. The electrode lead further comprises at least one electrode contact affixed to the cuff body, and at least one electrical conductor extending through the lead body between the at least one lead connector terminal and the electrode contact(s).

### BRIEF SUMMARY

One aspect is an electrical stimulation lead that includes a cuff having a cuff body having an exterior surface, an interior surface, and a circumference; longitudinal electrodes disposed on the interior surface of the cuff body, wherein each of the longitudinal electrodes has an aspect ratio of length/width of at least 20, wherein the longitudinal electrodes are divided into at least one set with each set including at least sixteen of the longitudinal electrodes spaced apart from each other in a circumferential arrangement round the circumference of the cuff body; and a longitudinal slit extending through the cuff body and further extending along an entire length of the cuff body, the longitudinal slit operable to receive a portion of a target nerve from a region outside of the cuff to within the cuff body. The electrical stimulation lead also includes a lead body coupled to the cuff and conductors extending through the lead body and the cuff with the conductors electrically coupled to the longitudinal electrodes.

In at least some aspects, the aspect ratio of each of the longitudinal electrodes is at least 50. In at least some aspects, each of the longitudinal electrodes has a width of no more than 100 µm. In at least some aspects, each of the longitudinal electrodes has a length of at least 1 mm. In at least some aspects, each of the at least one set includes at least 32 of the longitudinal electrodes spaced apart from each other in the circumferential arrangement around the circumference of the cuff body.

In at least some aspects, the cuff further includes at least one radial electrode extending around at least 75% of the circumference of the cuff body. In at least some aspects, the cuff further includes at least one set of radial electrodes, wherein each set of the radial electrodes includes at least two of the radial electrodes in a circumferential arrangement extending around at least 75% of the circumference of the cuff body.

Another aspect is an electrical stimulation lead that includes a cuff having a cuff body having an exterior surface, an interior surface, and a circumference; longitudinal electrodes disposed on the interior surface of the cuff body, wherein each of the longitudinal electrodes has a width of no more than 100 µm, wherein the longitudinal electrodes are divided into at least one set with each set including at least sixteen of the longitudinal electrodes spaced apart from each other in a circumferential arrangement round the circumference of the cuff body; and a longitudinal slit extending through the cuff body and further extending along an entire length of the cuff body, the longitudinal slit operable to receive a portion of a target nerve from a region outside of the cuff to within the cuff body. The electrical stimulation lead also includes a lead body coupled to the cuff and conductors extending through the lead body and the cuff with the conductors electrically coupled to the longitudinal electrodes.

In at least some aspects, the aspect ratio of each of the longitudinal electrodes is at least 50. In at least some aspects, each of the longitudinal electrodes has a length of at least 1 mm. In at least some aspects, each of the at least one set includes at least 32 of the longitudinal electrodes spaced apart from each other in the circumferential arrangement round the circumference of the cuff body.

In at least some aspects, the cuff further includes at least one radial electrode extending around at least 75% of the circumference of the cuff body. In at least some aspects, the cuff further includes at least one set of radial electrodes, wherein each set of the radial electrodes includes at least two of the radial electrodes in a circumferential arrangement extending around at least 75% of the circumference of the cuff body.

A further aspect is an electrical stimulation lead that includes a cuff having a cuff body having an exterior surface, an interior surface, and a circumference; longitudinal electrodes disposed on the interior surface of the cuff body, wherein the longitudinal electrodes are divided into at least one set with each set including at least sixteen or thirty-two of the longitudinal electrodes spaced apart from each other in a circumferential arrangement round the circumference of the cuff body; one or more radial electrodes extending solely, or in a combination of two or more of the radial electrodes (for example, when there are two or more radial electrodes), around at least 75% of the circumference of the cuff body; and a longitudinal slit extending through the cuff body and further extending along an entire length of the cuff body, the longitudinal slit operable to receive a portion of a target nerve from a region outside of the cuff to within the cuff body. The electrical stimulation lead also includes a lead body coupled to the cuff and conductors extending through the lead body and the cuff with conductors electrically coupled to the longitudinal and radial electrodes.

In at least some aspects, the aspect ratio of each of the longitudinal electrodes is at least 50. In at least some aspects, each of the longitudinal electrodes has a width of no more than 100 µm. In at least some aspects, each of the longitudinal electrodes has a length of at least 1 mm. In at least some aspects, each of the at least one set includes at least 32 of the longitudinal electrodes spaced apart from each other in the circumferential arrangement round the circumference of the cuff body.

In at least some aspects, the cuff further includes at least two sets of the radial electrodes, wherein each set of the radial electrodes includes at least one of the radial electrodes extending around at least 75% of the circumference of the cuff body. In at least some aspects, at least one of the sets of radial electrodes includes at least two of the radial electrodes extending, in combination, around at least 75% the circumference of the cuff body. In at least some aspects, the cuff further includes a cushioning layer disposed over the interior surface of the cuff body.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an electrical stimulation system that includes a lead electrically coupled to a control module;
FIG. 2A is a schematic view of one embodiment of the control module of FIG. 1 configured and arranged to electrically couple to an elongated device;
FIG. 2B is a schematic view of one embodiment of a lead extension configured and arranged to electrically couple the elongated device of FIG. 2A to the control module of FIG. 1;
FIG. 3 is a schematic perspective view of one embodiment of a cuff with two sets of sixteen longitudinal electrodes each and two radial electrodes;
FIG. 4 is a schematic perspective view of another embodiment of a cuff with four sets of sixteen longitudinal electrodes each and three radial electrodes;
FIG. 5 is a schematic perspective view of one embodiment of a cuff with four sets of sixteen longitudinal electrodes each and three sets of two radial electrodes each;
FIG. 6 is a schematic perspective view of one embodiment of a cuff with four sets of thirty-two longitudinal electrodes each and two radial electrodes;
FIG. 7 is a photograph of a cross-section of a portion of a vagus nerve;
FIG. 8 is a cross-sectional view of the cuff of FIG. 6 disposed around a portion of the vagus nerve; and
FIG. 9 is a schematic overview of one embodiment of components of an electrical stimulation arrangement according to an embodiment of the present invention.

### DETAILED DESCRIPTION

The present invention is directed to the area of implantable electrical stimulation systems and methods of making and using the systems. The present invention is also directed to implantable electrical stimulation cuff devices, as well as methods of making and using the same.

Suitable implantable electrical stimulation systems include, but are not limited to, a least one lead with one or more electrodes disposed along a distal end of the lead. Leads include, for example, percutaneous leads, paddle leads, and cuff leads. Examples of electrical stimulation systems with leads are found in, for example, U.S. Patents Nos. 6,181,969; 6,516,227; 6,609,029; 6,609,032; 6,741,892; 7,203,548; 7,244,150; 7,450,997; 7,596,414; 7,610,103; 7,672,734;7,761,165; 7,783,359; 7,792,590; 7,809,446; 7,949,395; 7,974,706; 6,175,710; 6,224,450; 6,271,094; 6,295,944; 6,364,278; and 6,391,985; U.S. Patent Applications Publication Nos. 2007/0150036; 2009/0187222; 2009/0276021; 2010/0076535; 2010/0268298; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; and 2013/0105071; and U.S. Patent Applications Serial Nos. 12/177,823 and 13/750,725.

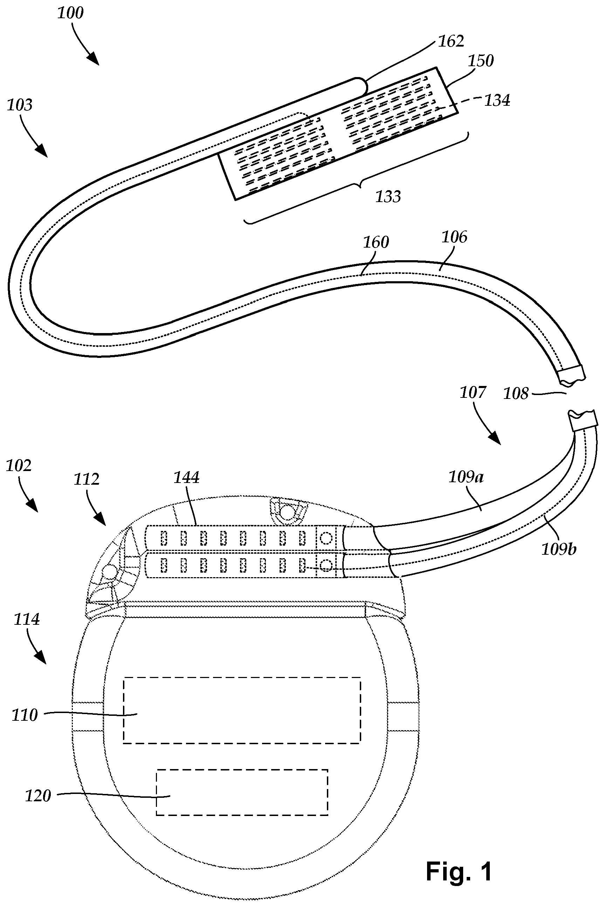

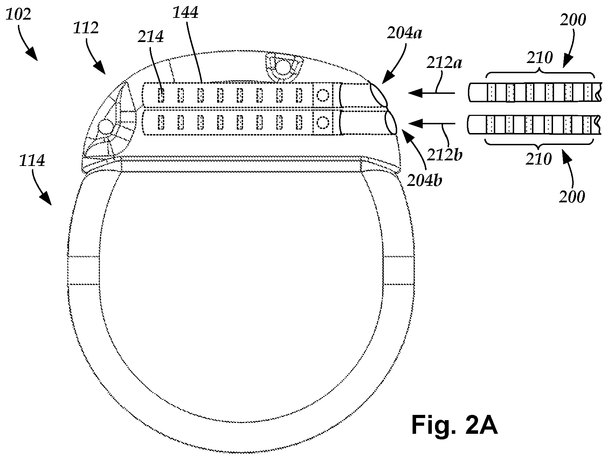

Figure 1 illustrates schematically one embodiment of an electrical stimulation system 100. The electrical stimulation system includes a control module (*e.g.,* a stimulator or pulse generator) 102 and a lead 103 coupleable to the control module 102. The lead 103 includes a mount 162 and a cuff 150. The lead 103 includes one or more lead bodies 106, an array of electrodes 133, such as electrode 134, and an array of terminals (*e.g.*, 210 in Figure 2A-2B) disposed within the cuff 150 attached to the one or more lead bodies 106. In at least some embodiments, the lead is isodiametric along at least a portion of the longitudinal length of the lead body 106. Figure 1 illustrates one lead 103 coupled to a control module 102. Other embodiments may include two, three, four, or more leads 103 coupled to the control module 102. In yet other embodiments, a lead 103 may be coupled to multiple control modules 102. For example, a lead with 64 electrodes may be coupled to two control modules 102 that are capable of handling 32 electrodes each.

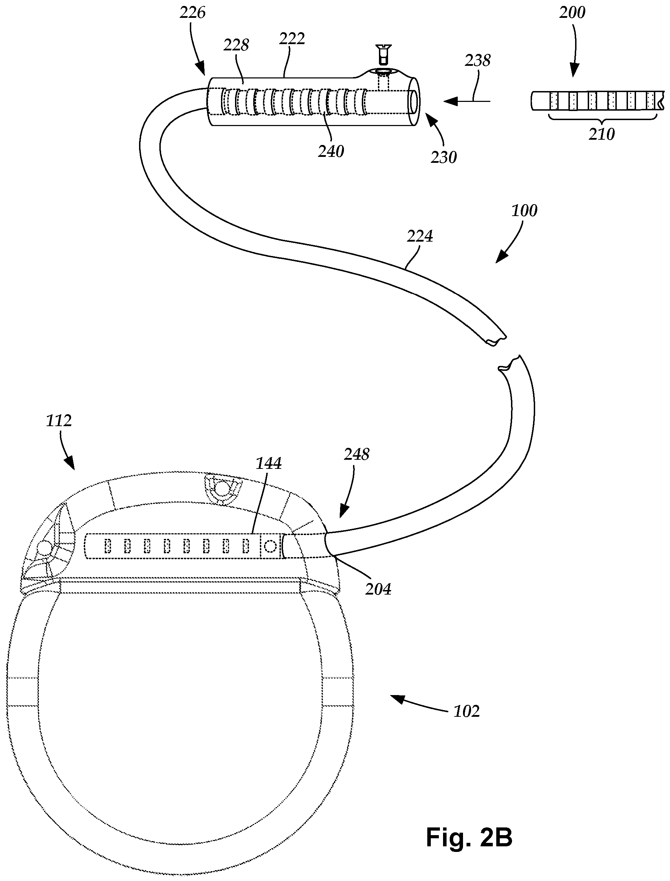

The lead 103 can be coupled to the control module 102 in any suitable manner. In at least some embodiments, the lead 103 couples directly to the control module 102. In at least some other embodiments, the lead 103 couples to the control module 102 via one or more intermediate devices (200 in Figures 2A-2B). For example, in at least some embodiments one or more lead extensions 224 (*see e.g.,* Figure 2B) can be disposed between the lead 103 and the control module 102 to extend the distance between the lead 103 and the control module 102. Other intermediate devices may be used in addition to, or in lieu of, one or more lead extensions including, for example, a splitter, an adaptor, or the like or combinations thereof. It will be understood that, in the case where the electrical stimulation system 100 includes multiple elongated devices disposed between the lead 103 and the control module 102, the intermediate devices may be configured into any suitable arrangement.

In Figure 1, the electrical stimulation system 100 is shown having a splitter 107 configured and arranged for facilitating coupling of the lead 103 to the control module 102. The splitter 107 includes a splitter connector 108 configured to couple to a proximal end of the lead 103, and one or more proximal tails 109a and 109b configured and arranged to couple to the control module 102 (or another splitter, a lead extension, an adaptor, or the like). The splitter 107 and splitter connector 108 may be part of the lead 103 or may be a separate component that attaches to the lead.

The control module 102 typically includes a connector housing 112 and a sealed electronics housing 114. Stimulation circuitry 110 and an optional power source 120 are disposed in the electronics housing 114. A control module connector 144 is disposed in the connector housing 112. The control module connector 144 is configured and arranged to make an electrical connection between the lead 103 and the stimulation circuitry 110 of the control module 102.

The electrical stimulation system or components of the electrical stimulation system, including the lead body 106 and the control module 102, are typically implanted into the body of a patient. The electrical stimulation system can be used for a variety of applications including, but not limited to, brain stimulation, neural stimulation, spinal cord stimulation, muscle stimulation, and the like.

The lead body 106 can be made of, for example, a non-conductive, biocompatible material such as, for example, silicone, polyurethane, polyetheretherketone ("PEEK"), epoxy, and the like or combinations thereof. The lead body 106 may be formed in the desired shape by any process including, for example, molding (including injection molding), casting, and the like. The non-conductive material typically extends from the distal end of the lead body 106 to the proximal end of the lead body 106.

Terminals (*e.g.*, 210 in Figures 2A-2B) are typically disposed along the proximal end of the lead body 106 of the electrical stimulation system 100 (as well as any splitters, lead extensions, adaptors, or the like) for electrical connection to corresponding connector contacts (*e.g.,* 214 and 240 in Figure 2B). The connector contacts are disposed in connectors (*e.g.,* 144 in Figures 1-2B; and 222 in Figure 2B) which, in turn, are disposed on, for example, the control module 102 (or a lead extension, a splitter, an adaptor, or the like). Electrically conductive wires 160, cables, or the like (only one of which is shown in Figure 1) extend from the terminals to the electrodes 134. Typically, one or more electrodes 134 are electrically coupled to each terminal. In at least some embodiments, each terminal is only connected to one electrode 134.

The electrically conductive wires ("conductors") 160 (only one of which is illustrated in Figure 1 for clarity) may be embedded in the non-conductive material of the lead body 106 or can be disposed in one or more lumens (not shown) extending along the lead body 106. In some embodiments, there is an individual lumen for each conductor. In other embodiments, two or more conductors extend through a lumen. There may also be one or more lumens (not shown) that open at, or near, the proximal end of the lead body 106, for example, for inserting a stylet to facilitate placement of the lead body 106 within a body of a patient. Additionally, there may be one or more lumens (not shown) that open at, or near, the distal end of the lead body 106, for example, for infusion of drugs or medication into the site of implantation of the lead body 106. In at least one embodiment, the one or more lumens are flushed continually, or on a regular basis, with saline, epidural fluid, or the like. In at least some embodiments, the one or more lumens are permanently or removably sealable at the distal end.

Figure 1 also illustrates a mount 162, part of the lead body 106, coupled to cuff 150. The conductors 160 (only one of which is illustrated in Figure 1 for clarity) from within the lead body 106 are received in the mount 162, which in turn is attached to the cuff 150 such that each conductor passes through the mount 162 for a direct electrical connection with one of the electrodes 134 (*e.g.,* one conductor is electrically connected with one electrode and so on). The mount 162 may be attached using a variety of means such as, but not limited to, molding or adhering the mount 162 to the cuff 150. In other embodiments, the conductors 160 from within the lead body 106 are electrically coupled to the electrodes 134 using jumper, intermediate or transition wires from the lead body 106 to the electrodes 134.

The mount 162 can be offset from the cuff 150, as illustrated in Figure 1, or in-line with the cuff or in any other suitable arrangement. Examples of cuff leads 103 can be found at U.S. Patents Nos. 7,596,414; 7,974,706; 8,423,157; 10,485,969; 10,493,269; 10,709,888; and 10,814,127; and U.S. Patent Application Publications Nos. 2017/0333692 and 2018/0154156.

Figure 2A is a schematic side view of one embodiment of a proximal end of one or more elongated devices 200 configured and arranged for coupling to one embodiment of the control module connector 144. The one or more elongated devices may include, for example, the lead body 106, one or more intermediate devices (*e.g.,* the lead extension 224 of Figure 2B, an adaptor, or the like or combinations thereof), or a combination thereof. Figure 2A illustrates two elongated devices 200 coupled to the control module 102. These two elongated devices 200 can be two tails as illustrated in Figure 1 or two different leads or any other combination of elongated devices.

The control module connector 144 defines at least one port into which a proximal end of the elongated device 200 can be inserted, as shown by directional arrow 212. In Figure 2A (and in other figures), the connector housing 112 is shown having two ports 204a and 204b. The connector housing 112 can define any suitable number of ports including, for example, one, two, three, four, five, six, seven, eight, or more ports.

The control module connector 144 also includes a plurality of connector contacts, such as connector contact 214, disposed within each port 204a and 204b. When the elongated device 200 is inserted into the ports 204a and 204b, the connector contacts 214 can be aligned with a plurality of terminals 210 disposed along the proximal end(s) of the elongated device(s) 200 to electrically couple the control module 102 to the electrodes (134 of Figure 1) disposed at a distal end of the lead 103. Examples of connectors in control modules are found in, for example, U.S. Patent No. 7,244,150 and 8,224,450.

Figure 2B is a schematic side view of another embodiment of the electrical stimulation system 100. The electrical stimulation system 100 includes a lead extension 224 that is configured and arranged to couple one or more elongated devices 200 (*e.g.,* the lead body 106, an adaptor, another lead extension, or the like or combinations thereof) to the control module 102. In Figure 2B, the lead extension 224 is shown coupled to a single port 204 defined in the control module connector 144. Additionally, the lead extension 224 is shown configured and arranged to couple to a single elongated device 200. In alternate embodiments, the lead extension 224 is configured and arranged to couple to multiple ports 204 defined in the control module connector 144, or to receive multiple elongated devices 200, or both.

A lead extension connector 222 is disposed on the lead extension 224. In Figure 2B, the lead extension connector 222 is shown disposed at a distal end 226 of the lead extension 224. The lead extension connector 222 includes a connector housing 228. The connector housing 228 defines at least one port 230 into which terminals 210 of the elongated device 200 can be inserted, as shown by directional arrow 238. The connector housing 228 also includes a plurality of connector contacts, such as connector contact 240. When the elongated device 200 is inserted into the port 230, the connector contacts 240 disposed in the connector housing 228 can be aligned with the terminals 210 of the elongated device 200 to electrically couple the lead extension 224 to the electrodes (134 of Figure 1) disposed along the lead (103 in Figure 1).

In at least some embodiments, the proximal end of the lead extension 224 is similarly configured and arranged as a proximal end of the lead 103 (or other elongated device 200). The lead extension 224 may include a plurality of electrically conductive wires (not shown) that electrically couple the connector contacts 240 to a proximal end 248 of the lead extension 224 that is opposite to the distal end 226. In at least some embodiments, the conductive wires disposed in the lead extension 224 can be electrically coupled to a plurality of terminals (not shown) disposed along the proximal end 248 of the lead extension 224. In at least some embodiments, the proximal end 248 of the lead extension 224 is configured and arranged for insertion into a connector disposed in another lead extension (or another intermediate device). In other embodiments (and as shown in Figure 2B), the proximal end 248 of the lead extension 224 is configured and arranged for insertion into the control module connector 144.

Conventional cuff leads include a cuff that wraps around a portion of a nerve with one or more electrodes arranged on the cuff. In many conventional cuff leads, the individual electrodes also wrap around at least a portion of the circumference of a nerve in a radial wrap arrangement. The radial wrap arrangement of the electrodes typically results in stimulation of a circumferential region of the nerve.

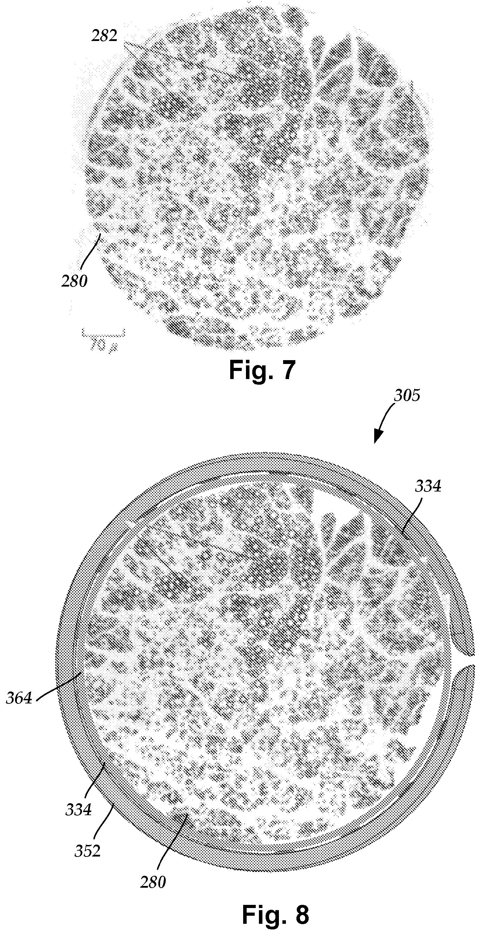

However, a nerve is not a monolithic biological construct, but, instead, the nerve is made of many fibers (which can be arranged in groups) that extend longitudinally along the nerve. Figure 7 is a cross-section of a portion of the vagus nerve 280 illustrating the many fibers 282 within the nerve. In some instances, it may be desirable to stimulate only one fiber or a group of fibers.

Electrodes in a radial wrap arrangement generally cannot selectively stimulate fibers or groups of fibers, but, instead, such electrodes stimulate many fibers due to extending around the circumference of the nerve. In addition, such electrodes may produce unwanted side effects as multiple nerve fibers are stimulated. For example, a cuff lead with a cuff around the vagus nerve can have wide ranging effects when stimulating the vagus nerve because the different fibers connect to many parts of the body.

As described further herein, a cuff lead can include a cuff body that wraps around a nerve and includes longitudinal electrodes distributed around the circumference of the cuff body. In at least some embodiments, these longitudinal electrodes permit the targeting of selected longitudinal regions along the circumference of the cuff body. In at least some embodiments, there are at least 16, 20, 25, 28, 32, 36, 40, 48, 50, 64, 80, 100, 120, 128, 150, 200, 250, 256, or more longitudinal electrodes arranged in a set around the circumference of the cuff body and there may be one, two, three, or more sets of longitudinal electrodes that are spaced apart longitudinally from each other along the cuff body.

In at least some embodiments, the cuff may also include one or more radial electrodes that can be used as a counter-electrode to one or more selected longitudinal electrodes. In at least some embodiments, one or more of the longitudinal electrodes can be used as a cathode(s) and one or more of the radial electrodes can be used as an anode(s). Any other suitable selection of cathode(s) or anode(s) from the longitudinal or radial electrodes can be used.

In at least some embodiments, the longitudinal electrodes can be used to selectively stimulate a nerve fiber or a set of nerve fibers. For example, a cuff lead with a cuff around the vagus nerve may be used to selectively stimulate immunomodulation fibers without stimulating (or with reduced or subthreshold stimulation of) cardiovascular fibers or somatotopic fibers in the nerve. For example, the immunomodulation fibers may be used to enhance, decrease, or halt signaling to or from the brain. In at least some embodiments, a cuff lead with longitudinal electrodes can be used to selectively provide fiber or fascicular stimulation.

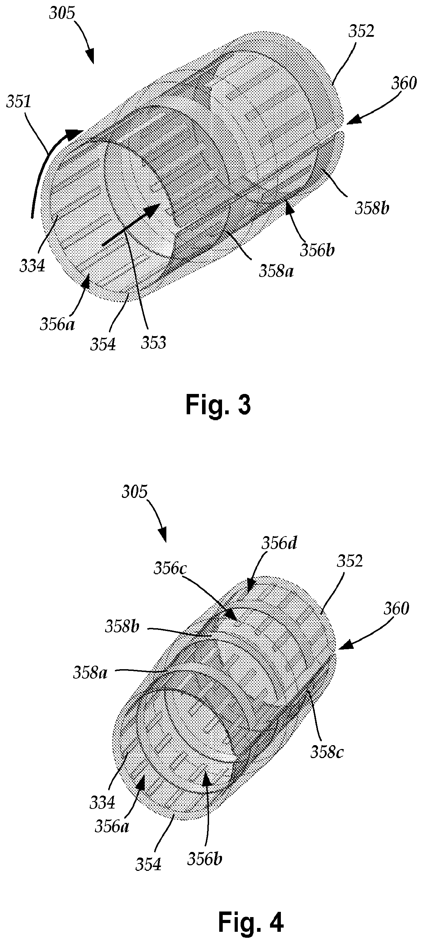

Figure 3 illustrates one embodiment of a cuff 350 of a cuff lead 103 (Figure 1). The cuff 350 includes a cuff body 352 with longitudinal electrodes 334 disposed on an interior surface 354 of the cuff body and arranged around the circumference of the cuff body in two sets 356a, 356b. In the illustrated embodiment, each set 356a, 356b includes sixteen longitudinal electrodes 334. Any other suitable number of electrodes can be used including, but not limited to, 16, 20, 25, 28, 32, 36, 40, 48, 50, 64, 80, 100, 120, 128, 150, 200, 250, 256, or more longitudinal electrodes. A cuff lead can have one, two, three, four, or more sets of longitudinal electrodes 334. The number of longitudinal electrodes 334 in a set can be the same for each set or can differ. In the illustrated embodiment, the longitudinal electrodes 334 of each set are aligned longitudinally with electrodes of the other set. In other embodiments, the longitudinal electrodes 334 of each set can be staggered or unaligned with the electrodes of the other set.

In addition, the cuff 350 includes two radial electrodes 358a, 358b that wrap around at least 75%, 80%, 90%, or 95% of the circumference of the cuff body 352. The cuff 350 also defines a slit 360 that extends the longitudinal length of the cuff body 352 so that the nerve can be loaded into the interior 362 of the cuff body by opening the slit to fit the cuff body over the nerve. The slit 360 is opened or initially sized to allow the target nerve (not shown) to be slipped, inserted, fed, or otherwise received into the cuff 350 such that the cuff 350 wraps around the target nerve. In at least some embodiments, the slit 360 allows the cuff 350 to be easily moved over and around the target nerve or relative to the target nerve whether rotationally or transitionally.

The electrodes 334, 358a, 358b can be formed using any conductive, biocompatible material. Examples of suitable materials include metals, alloys, conductive polymers, conductive carbon, and the like, as well as combinations thereof. In at least some embodiments, one or more of the electrodes 334 are formed from one or more of: platinum, platinum alloys such as platinum iridium, palladium alloys such as palladium rhodium, titanium, titanium alloys, nickel alloys, cobalt alloys, nickel/cobalt alloys, stainless steel, tantalum, conductive carbon, conductive plastics, epoxy or other adhesive filled with metallic powder, Nitinol^{™}, or the like or any combination thereof. The electrodes 334, 358a, 358b can be formed by any suitable process including, but not limited to, machining, molding (for example, powdered metal molding), photolithography, additive techniques, stamping, or the like or any combination thereof.

In at least some embodiments, the electrodes 334, 358a, 358b have a contact surface that is flush or slightly protruding (for example, no more than 200, 100, or 50 µm from the cuff body 352 which, at least in some circumstances, may reduce or eliminate physical pressure on the nerve. It will be recognized that the electrodes can be used to provide electrical stimulation or to sense electrical signals from tissue or any combination thereof.

In at least some embodiments, the longitudinal electrodes 334 have a width of no more than 100, 75, 50, 40, 30, or 25 micrometers (µm) and a length of at least 1, 2, 3, 4, 5, 7, or more millimeters (mm). The width of the longitudinal electrodes corresponds to a distance in the circumferential direction 351 around the cuff body. In at least some embodiments, the length of the longitudinal electrodes 334 is no more than 10 mm. The length of the longitudinal electrodes corresponds to a distance along the longitudinal direction 353 of the cuff body. In at least some embodiments, the longitudinal electrodes 334 have an aspect ratio (length/width) or at least 20, 40, 50, 80, 100, 150, 200, or more. In at least some embodiments, each of the electrodes 334 has the same width, length, and aspect ratio. In other embodiments, the electrodes 334 can have different widths, lengths, or aspect ratios with electrodes of a set have the same or different widths, lengths, or aspect ratios within the set or between sets.

In at least some embodiments, the longitudinal electrodes 334 are rectangular or rectangular with rounded corners. Any other suitable shape can be used for the longitudinal electrodes including, but not limited to, oblong, oval, modified rectangular with one or more sides (or portions of sides) that are curved, or the like or any combination thereof. The length and width measurements described in the preceding paragraph correspond to the longest or widest portion of the electrode 334. For example, for an oval electrode, the length along the major axis of the oval corresponds to the length measurement and the length along the minor axis corresponds to the width measurement.

The narrow width of the longitudinal electrodes 334 can facilitate the ability to select particular fibers or groups of fibers in the nerve and steer the stimulation to the selected fiber or group of fibers. The number of longitudinal electrodes 334 in each set can further enhance the fiber selectivity with increasing numbers of longitudinal electrodes 334 providing more selectivity. Stimulation can be performed using one or more of the longitudinal electrodes 334. The selection of an appropriate radial electrode 358a, 358b (or one or more of the longitudinal electrodes 334) as the counter-electrode can further enhance steering of the stimulation to the selected fiber or group of fibers.

The cuff body 352 can be formed of any suitable biocompatible and biostable non-conductive material including, but not limited to, polymer materials such as silicone, polyurethane, polyetheretherketone ("PEEK"), epoxy, or the like or any combination thereof. In at least some embodiments, the cuff body 352 can have a circular, oval, or any other suitable cross-sectional shape and, at least in some embodiments, may be sufficiently flexible to alter the cross-sectional shape to accommodate the nerve. In at least some embodiments, the electrodes 334, 358a, 358b can be molded with the cuff body 352 or formed by techniques such as etching or ablation of conductive layers, films, or the like. In at least some embodiments, the cuff body 352 has an inner diameter (which can correspond to the largest diameter of a non-circular cuff body) in a range of 0.5 to 5 mm or in a range of 1 to 3 mm. In at least some embodiments, the cuff body 352 has a length of at least 5, 10, or 20 mm.

In at least some embodiments, the cuff body 352 can be formed using any suitable technique including, but not limited to, molding, casting, formed in a sheet and then shaped using adhesive as a binder, formed flat and shaped using heat, formed flat and attached to a cuff-shaped scaffold, pressed or extruded into the cuff shape, or the like or any combination thereof. In at least some embodiments, the electrodes 334 can be attached to the cuff body 352 using any suitable technique including, but not limited to, attaching with adhesive, molding (for example, insert molding) into the cuff body, using heat to adhere the electrodes to the cuff body, heating and pressing the electrodes into the cuff body, depositing electrode material on the cuff body and using photolithography and etching, or the like or any combination thereof.

In at least some embodiments, the interior surface 354 of the cuff body 352 can be coated with a cushioning layer 364 (Figure 8) to act as a cushion to reduce damage to the nerve. Examples of materials for the cushioning layer 364 include, but are not limited to, paraffin, a combination of isotonic saline and artificial cerebrospinal fluid, or the like or any combination thereof. The cushioning layer 364 is made of a material that permits flow of current from the electrodes 334 to the nerve through the cushioning layer.

In at least some embodiments, once the cuff 350 has been placed in a desired position relative to the target nerve, the edges of the cuff body 352 defining the slit360 can be sutured to capture the target nerve without undesirably compressing the target nerve. In at least some embodiments, suture holes (not shown) are optionally incorporated into the edges of the cuff 350 to allow for closing or partially closing the cuff 350 around the target nerve.

Figure 4 illustrates another embodiment of a cuff 350 with a cuff body 352 and longitudinal electrodes 334 arranged in four groups 356a, 356b, 356c, 356d with sixteen electrodes in each group. The cuff 350 includes three radial electrodes 358a, 358b, 358c.

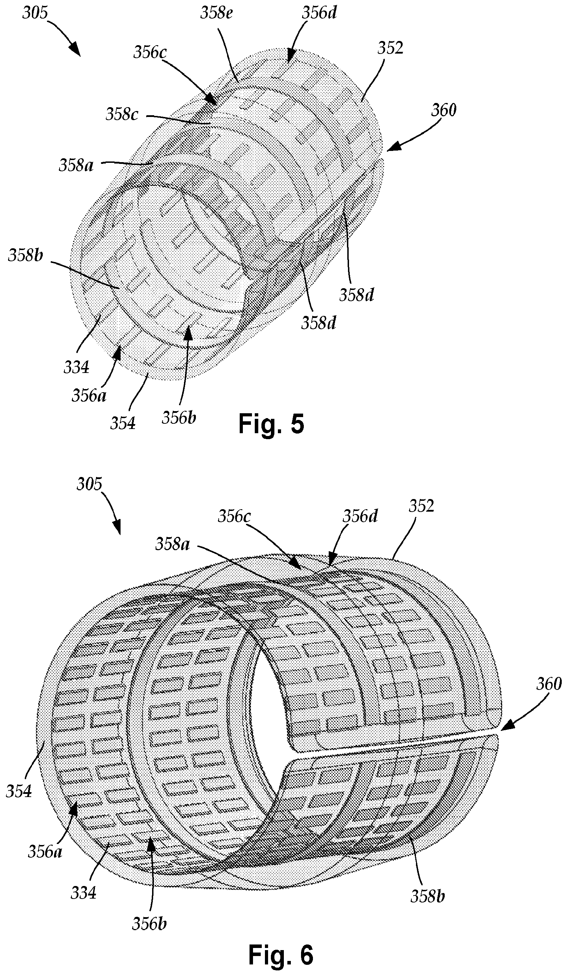

Figure 5 illustrates another embodiment of a cuff 350 with a cuff body 352 and longitudinal electrodes 334 arranged in four groups 356a, 356b, 356c, 356d with sixteen electrodes in each group. The cuff 350 includes six radial electrodes 358a, 358b, 358c, 358d, 358e, 358f that each extend around less than half the circumference of the cuff body 352 (for example, at least 25%, 30%, 33%, 40%, 45%, or 48% of the circumference of the cuff body) with two of these radial electrodes disposed in each of three sets. It will be understood that other arrangement can include, for example, three, four, six or more radial electrodes (or any other number of radial electrodes) per set. The radial electrodes of a set can extend a same amount around the circumference of the cuff body 352 or can extend by different amounts around the circumference of the cuff body. Each set can be identical, or the sets can have a different arrangement of radial electrodes. In at least some embodiments, the radial electrodes of a set, in combination, extend around at least 75%, 80%, 90%, or 95% of the circumference of the cuff body 352.

Figure 6 illustrates yet another embodiment of a cuff 350 with a cuff body 352 and longitudinal electrodes 334 arranged in four groups 356a, 356b, 356c, 356d with 32 electrodes in each group. The cuff 350 includes two radial electrodes 358a, 358b.

Figure 8 illustrates a cross-section of the cuff 350 of Figure 6 disposed around the vagus nerve 280 with the longitudinal electrodes 334 arranged around the circumference of the cuff and vagus nerve. Optionally, the cushioning layer 364 is disposed between the cuff 350/electrodes 334 and the nerve 280.

The cuff lead 103 (Figure 1) can be coupled to one or more control modules 102 (Figure 1). When the cuff lead 103 has many longitudinal electrodes 334, multiple control modules 102 may be used to independently control the longitudinal electrodes 334. Additionally or alternatively, multiplexing techniques and arrangements can be used to provide stimulation to selected longitudinal electrodes 334. Multiplexing arrangements may be part of the control module 102, cuff lead 103, or a separate module or the like or any combination thereof. Examples of multiplexing and of independent control and delivery of stimulation through selected electrodes can be found in U.S. Patents Nos. 8,423,154; 8,606,362; 8,620,436; 9,308,383; 9,568,053; 10,350,413; and 10,537,741; and U.S. Patent Application Publications Nos. 2018/0071520 and 2019/0083796.

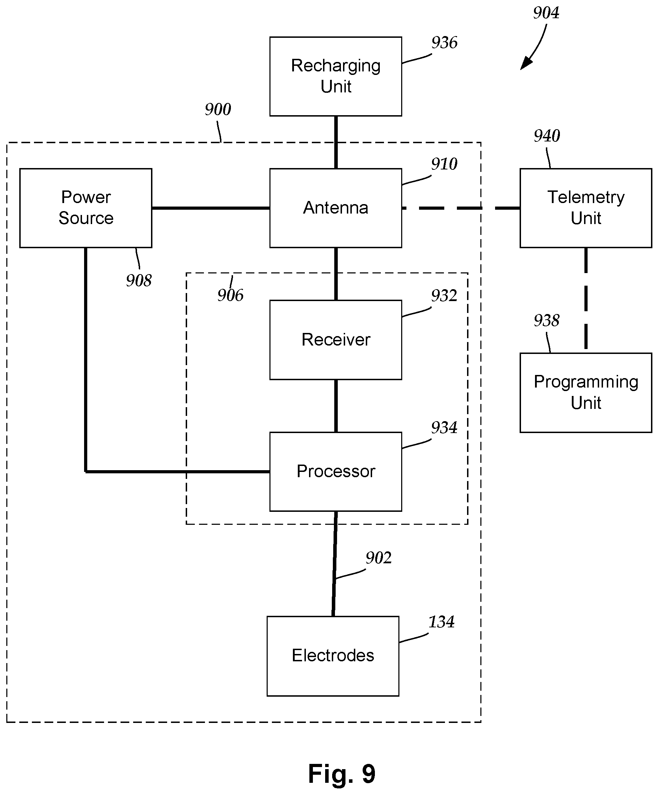

Figure 9 is a schematic overview of one embodiment of components of an electrical stimulation arrangement 904 that includes an electrical stimulation system 900 with a lead 902, stimulation circuitry 906, a power source 908, and an antenna 910. The electrical stimulation system can be, for example, any of the electrical stimulation systems described above. It will be understood that the electrical stimulation arrangement can include more, fewer, or different components and can have a variety of different configurations including those configurations disclosed in the stimulator references cited herein.

If the power source 908 is a rechargeable battery or chargeable capacitor, the power source may be recharged/charged using the antenna 910, if desired. Power can be provided for recharging/charging by inductively coupling the power source 908 through the antenna 910 to a recharging unit 936 external to the user. Examples of such arrangements can be found in the references identified above.

In at least some embodiments, electrical current is emitted by the electrodes (such as electrodes 134 in Figure 1) on the lead 902 to stimulate nerve fibers, muscle fibers, or other body tissues near the electrical stimulation system. The stimulation circuitry 906 can include, among other components, a processor 934 and a receiver 932. The processor 934 is generally included to control the timing and electrical characteristics of the electrical stimulation system. For example, the processor 934 can, if desired, control one or more of the timing, frequency, strength, duration, and waveform of the pulses. In addition, the processor 934 can select which electrodes can be used to provide stimulation, if desired. In some embodiments, the processor 934 selects which electrode(s) are cathodes and which electrode(s) are anodes. In some embodiments, the processor 934 is used to identify which electrodes provide the most useful stimulation of the desired tissue.

Any processor can be used and can be as simple as an electronic device that, for example, produces pulses at a regular interval or the processor can be capable of receiving and interpreting instructions from an external programming unit 938 that, for example, allows modification of pulse characteristics. In the illustrated embodiment, the processor 934 is coupled to a receiver 932 which, in turn, is coupled to the antenna 910. This allows the processor 934 to receive instructions from an external source to, for example, direct the pulse characteristics and the selection of electrodes, if desired.

In at least some embodiments, the antenna 910 is capable of receiving signals (*e.g.,* RF signals) from an external telemetry unit 940 that is programmed by the programming unit 938. The programming unit 938 can be external to, or part of, the telemetry unit 940. The telemetry unit 940 can be a device that is worn on the skin of the user or can be carried by the user and can have a form similar to a pager, cellular phone, or remote control, if desired. As another alternative, the telemetry unit 940 may not be worn or carried by the user but may only be available at a home station or at a clinician's office. The programming unit 938 can be any unit that can provide information to the telemetry unit 940 for transmission to the electrical stimulation system 900. The programming unit 938 can be part of the telemetry unit 940 or can provide signals or information to the telemetry unit 940 via a wireless or wired connection. One example of a suitable programming unit is a computer operated by the user or clinician to send signals to the telemetry unit 940.

The signals sent to the processor 934 via the antenna 910 and the receiver 932 can be used to modify or otherwise direct the operation of the electrical stimulation system 900. For example, the signals may be used to modify the pulses of the electrical stimulation system such as modifying one or more of pulse duration, pulse frequency, pulse waveform, and pulse strength. The signals may also direct the electrical stimulation system 900 to cease operation, to start operation, to start charging the battery, or to stop charging the battery.

Optionally, the electrical stimulation system 900 may include a transmitter (not shown) coupled to the processor 934 and the antenna 910 for transmitting signals back to the telemetry unit 940 or another unit capable of receiving the signals. For example, the electrical stimulation system 900 may transmit signals indicating whether the electrical stimulation system 900 is operating properly or not or indicating when the battery needs to be charged or the level of charge remaining in the battery. The processor 934 may also be capable of transmitting information about the pulse characteristics so that a user or clinician can determine or verify the characteristics.

The above specification provides a description of the structure, manufacture, and use of the invention. Since many embodiments of the invention can be made without departing from the scope of the invention, the invention resides in the claims

## Claims

1. An electrical stimulation lead (103) comprising:
a cuff (150; 305) comprising
a cuff body (352) having an exterior surface, an interior surface (354), and a circumference,
a plurality of longitudinal electrodes (334) disposed on the interior surface of the cuff body (352), wherein each of the longitudinal electrodes (334) has an aspect ratio of length/width of at least 20, wherein the plurality of longitudinal electrodes (334) is divided into at least one set with each set comprising at least sixteen of the longitudinal electrodes spaced apart from each other in a circumferential arrangement round the circumference of the cuff body (352), and
a longitudinal slit (360) extending through the cuff body (352) and further extending along an entire length of the cuff body (352), the longitudinal slit (360) operable to receive a portion of a target nerve from a region outside of the cuff to within the cuff body (352);
a lead body (106) coupled to the cuff (150; 305); and
a plurality of conductors (160) extending through the lead body (106) and the cuff (150; 305) with the plurality of conductors (160) electrically coupled to the longitudinal electrodes (334).

2. The electrical stimulation lead of claim 1, wherein each of the longitudinal electrodes has a width of no more than 100 µm.

3. The electrical stimulation lead of any one of claims 1 or 2, wherein the cuff further comprises at least one radial electrode extending around at least 75% of the circumference of the cuff body.

4. The electrical stimulation lead of any one of claims 1 to 3, wherein the cuff further comprises at least one set of radial electrodes, wherein each set of the radial electrodes comprises at least two of the radial electrodes in a circumferential arrangement extending around at least 75% of the circumference of the cuff body.

5. An electrical stimulation lead (103) comprising:
a cuff (150; 305) comprising
a cuff body (352) having an exterior surface, an interior surface (354), and a circumference,
a plurality of longitudinal electrodes (334) disposed on the interior surface of the cuff body (352), wherein each of the longitudinal electrodes (334) has a width of no more than 100 µm, wherein the plurality of longitudinal electrodes (334) is divided into at least one set with each set comprising at least sixteen of the longitudinal electrodes spaced apart from each other in a circumferential arrangement round the circumference of the cuff body (352), and
a longitudinal slit (360) extending through the cuff body (352) and further extending along an entire length of the cuff body (352), the longitudinal slit (360) operable to receive a portion of a target nerve from a region outside of the cuff (150; 305) to within the cuff body (352);
a lead body (106) coupled to the cuff (150; 305); and
a plurality of conductors (160) extending through the lead body (106) and the cuff (150; 305) with the plurality of conductors (160) electrically coupled to the longitudinal electrodes (334).

6. The electrical stimulation lead of claim 5, wherein the cuff further comprises at least one radial electrode extending around at least 75% of the circumference of the cuff body.

7. The electrical stimulation lead of any one of claims 5 or 6, wherein the cuff further comprises at least one set of radial electrodes, wherein each set of the radial electrodes comprises at least two of the radial electrodes in a circumferential arrangement extending around at least 75% of the circumference of the cuff body.

8. The electrical stimulation lead of any one of claims 1 to 7, wherein an aspect ratio of length/width of each of the longitudinal electrodes is at least 50.

9. The electrical stimulation lead of any one of claims 1 to 8, further comprising a cushioning layer disposed over the interior surface of the cuff body.

10. The electrical stimulation lead of claim any one of claims 1 to 9, wherein each of the longitudinal electrodes has a length of at least 1 mm.

11. The electrical stimulation lead of any one of claims 1 to 10, wherein each of the at least one set of longitudinal electrodes comprises at least 32 of the longitudinal electrodes spaced apart from each other in the circumferential arrangement around the circumference of the cuff body.

## Patentansprüche

1. Elektrische Stimulationsleitung (103), die aufweist:
eine Manschette (150; 305) mit
einem Manschettenkörper (352), der eine Außenfläche, eine Innenfläche (354) und einen Umfang hat,
mehreren Längselektroden (334), die auf der Innenfläche des Manschettenkörpers (352) angeordnet sind, wobei jede der Längselektroden (334) ein Aspektverhältnis von Länge/Breite von mindestens 20 hat, wobei die mehreren Längselektroden (334) in mindestens einen Satz aufgeteilt sind, wobei jeder Satz mindestens sechzehn der Längselektroden aufweist, die in einer Umfangsanordnung um den Umfang des Manschettenkörpers (352) voneinander beabstandet sind, und
einem Längsschlitz (360), der sich durch den Manschettenkörper (352) erstreckt und sich ferner entlang einer Gesamtlänge des Manschettenkörpers (352) erstreckt, wobei der Längsschlitz (360) so betriebsfähig ist, dass er einen Abschnitt eines Zielnervs von einem Bereich außerhalb der Manschette im Manschettenkörper (352) aufnimmt;
einen Leitungskörper (106), der mit der Manschette (150; 305) gekoppelt ist; und
mehrere Leiter (160), die sich durch den Leitungskörper (106) und die Manschette (150; 305) erstrecken, wobei die mehreren Leiter (160) mit den Längselektroden (334) elektrisch gekoppelt sind.

2. Elektrische Stimulationsleitung nach Anspruch 1, wobei jede der Längselektroden eine Breite von höchstens 100 µm hat.

3. Elektrische Stimulationsleitung nach Anspruch 1 oder 2, wobei die Manschette ferner mindestens eine Radialelektrode aufweist, die sich um mindestens 75 % des Umfangs des Manschettenkörpers erstreckt.

4. Elektrische Stimulationsleitung nach einem der Ansprüche 1 bis 3, wobei die Manschette ferner mindestens einen Satz von Radialelektroden aufweist, wobei jeder Satz der Radialelektroden mindestens zwei der Radialelektroden in einer Umfangsanordnung aufweist, die sich um mindestens 75 % des Umfangs des Manschettenkörpers erstreckt.

5. Elektrische Stimulationsleitung (103), die aufweist:
eine Manschette (150; 305) mit
einem Manschettenkörper (352), der eine Außenfläche, eine Innenfläche (354) und einen Umfang hat,
mehreren Längselektroden (334), die auf der Innenfläche des Manschettenkörpers (352) angeordnet sind, wobei jede der Längselektroden (334) eine Breite von höchstens 100 µm hat, wobei die mehreren Längselektroden (334) in mindestens einen Satz aufgeteilt sind, wobei jeder Satz mindestens sechzehn der Längselektroden aufweist, die in einer Umfangsanordnung um den Umfang des Manschettenkörpers (352) voneinander beabstandet sind, und
einem Längsschlitz (360), der sich durch den Manschettenkörper (352) erstreckt und sich ferner entlang einer Gesamtlänge des Manschettenkörpers (352) erstreckt, wobei der Längsschlitz (360) so betriebsfähig ist, dass er einen Abschnitt eines Zielnervs von einem Bereich außerhalb der Manschette (150; 305) im Manschettenkörper (352) aufnimmt;
einen Leitungskörper (106), der mit der Manschette (150; 305) gekoppelt ist; und
mehrere Leiter (160), die sich durch den Leitungskörper (106) und die Manschette (150; 305) erstrecken, wobei die mehreren Leiter (160) mit den Längselektroden (334) elektrisch gekoppelt sind.

6. Elektrische Stimulationsleitung nach Anspruch 5, wobei die Manschette ferner mindestens eine Radialelektrode aufweist, die sich um mindestens 75 % des Umfangs des Manschettenkörpers erstreckt.

7. Elektrische Stimulationsleitung nach Anspruch 5 oder 6, wobei die Manschette ferner mindestens einen Satz von Radialelektroden aufweist, wobei jeder Satz der Radialelektroden mindestens zwei der Radialelektroden in einer Umfangsanordnung aufweist, die sich um mindestens 75 % des Umfangs des Manschettenkörpers erstreckt.

8. Elektrische Stimulationsleitung nach einem der Ansprüche 1 bis 7, wobei ein Aspektverhältnis von Länge/Breite jeder der Längselektroden mindestens 50 beträgt.

9. Elektrische Stimulationsleitung nach einem der Ansprüche 1 bis 8, ferner mit einer Polsterschicht, die über der Innenfläche des Manschettenkörpers angeordnet ist.

10. Elektrische Stimulationsleitung nach einem der Ansprüche 1 bis 9, wobei jede der Längselektroden eine Länge von mindestens 1 mm hat.

11. Elektrische Stimulationsleitung nach einem der Ansprüche 1 bis 10, wobei jeder des mindestens einen Satzes von Längselektroden mindestens 32 der Längselektroden aufweist, die in der Umfangsanordnung um den Umfang des Manschettenkörpers voneinander beabstandet sind.

## Revendications

1. Fil de stimulation électrique (103) comprenant :
un manchon (150 ; 305) comprenant
un corps de manchon (352) ayant une surface extérieure, une surface intérieure (354) et une circonférence,
une pluralité d'électrodes longitudinales (334) disposées sur la surface intérieure du corps de manchon (352), dans lequel chacune des électrodes longitudinales (334) présente un rapport d'aspect de longueur/largeur d'au moins 20, dans lequel la pluralité d'électrodes longitudinales (334) est divisée en au moins un ensemble, chaque ensemble comprenant au moins seize des électrodes longitudinales espacées les unes des autres dans un agencement circonférentiel autour de la circonférence du corps de manchon (352), et
une fente longitudinale (360) s'étendant à travers le corps de manchon (352) et s'étendant en outre le long d'une longueur entière du corps de manchon (352), la fente longitudinale (360) servant à recevoir une portion d'un nerf cible d'une région à l'extérieur du manchon jusqu'à l'intérieur du corps de manchon (352) ;
un corps de fil (106) couplé au manchon (150 ; 305) ; et
une pluralité de conducteurs (160) s'étendant à travers le corps de fil (106) et le manchon (150 ; 305), la pluralité de conducteurs (160) étant couplés électriquement aux électrodes longitudinales (334).

2. Fil de stimulation électrique selon la revendication 1, dans lequel chacune des électrodes longitudinales a une largeur ne dépassant pas 100 µm.

3. Fil de stimulation électrique selon l'une quelconque des revendications 1 ou 2, dans lequel le manchon comprend en outre au moins une électrode radiale s'étendant autour d'au moins 75 % de la circonférence du corps de manchon.

4. Fil de stimulation électrique selon l'une quelconque des revendications 1 à 3, dans lequel le manchon comprend en outre au moins un ensemble d'électrodes radiales, dans lequel chaque ensemble des électrodes radiales comprend au moins deux des électrodes radiales dans un agencement circonférentiel s'étendant autour d'au moins 75 % de la circonférence du corps de manchon.

5. Fil de stimulation électrique (103) comprenant :
un manchon (150 ; 305) comprenant
un corps de manchon (352) ayant une surface extérieure, une surface intérieure (354) et une circonférence,
une pluralité d'électrodes longitudinales (334) disposées sur la surface intérieure du corps de manchon (352), dans lequel chacune des électrodes longitudinales (334) a une largeur ne dépassant pas 100 µm, dans lequel la pluralité d'électrodes longitudinales (334) est divisée en au moins un ensemble, chaque ensemble comprenant au moins seize des électrodes longitudinales espacées les unes des autres dans un agencement circonférentiel autour de la circonférence du corps de manchon (352), et
une fente longitudinale (360) s'étendant à travers le corps de manchon (352) et s'étendant en outre le long d'une longueur entière du corps de manchon (352), la fente longitudinale (360) servant à recevoir une portion d'un nerf cible d'une région à l'extérieur du manchon (150 ; 305) jusqu'à l'intérieur du corps de manchon (352) ;
un corps de fil (106) couplé au manchon (150 ; 305) ; et
une pluralité de conducteurs (160) s'étendant à travers le corps de fil (106) et le manchon (150 ; 305), la pluralité de conducteurs (160) étant couplés électriquement aux électrodes longitudinales (334).

6. Fil de stimulation électrique selon la revendication 5, dans lequel le manchon comprend en outre au moins une électrode radiale s'étendant autour d'au moins 75 % de la circonférence du corps de manchon.

7. Fil de stimulation électrique selon l'une quelconque des revendications 5 ou 6, dans lequel le manchon comprend en outre au moins un ensemble d'électrodes radiales, dans lequel chaque ensemble des électrodes radiales comprend au moins deux des électrodes radiales dans un agencement circonférentiel s'étendant autour d'au moins 75 % de la circonférence du corps de manchon.

8. Fil de stimulation électrique selon l'une quelconque des revendications 1 à 7, dans lequel un rapport d'aspect de longueur/largeur de chacune des électrodes longitudinales est d'au moins 50.

9. Fil de stimulation électrique selon l'une quelconque des revendications 1 à 8, comprenant en outre une couche d'amortissement disposée sur la surface intérieure du corps de manchon.

10. Fil de stimulation électrique selon l'une quelconque des revendications 1 à 9, dans lequel chacune des électrodes longitudinales a une longueur d'au moins 1 mm.

11. Fil de stimulation électrique selon l'une quelconque des revendications 1 à 10, dans lequel chacune de l'au moins un ensemble d'électrodes longitudinales comprend au moins 32 des électrodes longitudinales espacées les unes des autres dans l'agencement circonférentiel autour de la circonférence du corps de manchon.
